(19) Europäisches Patentamt
European Patent Office
Office européen des brevets

(11) EP 1 014 960 B1

(12) FASCICULE DE BREVET EUROPEEN

(45) Date de publication et mention de la délivrance du brevet:
**05.02.2003 Bulletin 2003/06**

(21) Numéro de dépôt: **98928407.0**

(22) Date de dépôt: **02.06.1998**

(51) Int Cl.$^7$: **A61K 31/15**, A61P 11/00

(86) Numéro de dépôt international:
**PCT/FR98/01100**

(87) Numéro de publication internationale:
**WO 99/000119 (07.01.1999 Gazette 1999/01)**

# (54) UTILISATION D'UN ANTAGONISTE SPECIFIQUE DES RECEPTEURS 5HT2 POUR LA PREPARATION DE MEDICAMENTS UTILES DANS LE TRAITEMENT DU SYNDROME D'APNEE DU SOMMEIL

VERWENDUNG EINES 5HT2 REZEPTOR-ANTAGONISTEN ZUR BEHANDLUNG VON SCHLAFAPNOE

USE OF A SPECIFIC ANTAGONIST OF 5HT2 RECEPTORS FOR PREPARING MEDICINES USEFUL FOR TREATING SLEEP-DISORDERED BREATHING

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **26.06.1997 FR 9707998**

(43) Date de publication de la demande:
**05.07.2000 Bulletin 2000/27**

(73) Titulaire: **SANOFI-SYNTHELABO**
**75013 Paris (FR)**

(72) Inventeur: **CATTELIN, Françoise**
**F-75016 Paris (FR)**

(74) Mandataire: **Thouret-Lemaitre, Elisabeth et al**
**Sanofi-Synthélabo**
**Service Brevets**
**174, avenue de France**
**75013 Paris (FR)**

(56) Documents cités:
**EP-A- 0 373 998** **EP-A- 0 518 767**

- **DATABASE BIOSIS BIOSCIENCES INFORMATION SERVICE, PHILADELPHIA, PA, US AN: 92:194860, M. YOSHIOKA ET AL: "Pharmacological Characterization of 5-Hydroxytryptamine-induced Apnea in the Rat" XP002060471**
- **DATABASE BIOSIS BIOSCIENCES INFORMATION SERVICE, PHILADELPHIA, PA, US AN: 96:131832, S. C. VEASEY: "The effect of serotonin antagonists in an animal model of sleep-disordered breathing" XP002060472**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne une nouvelle utilisation d'un antagoniste spécifique des récepteurs $5HT_{2A}$.

Le 1-(2-fluorophényl)-3-(4-hydroxyphényl)-prop-2-èn-1-one-O-2-diméthylamino éthyl)-oxime de formule (I) et ses sels pharmaceutiquement acceptables, sont décrits dans le brevet européen 0 373 998 B1 comme des antagonistes des récepteurs $5HT_2$ :

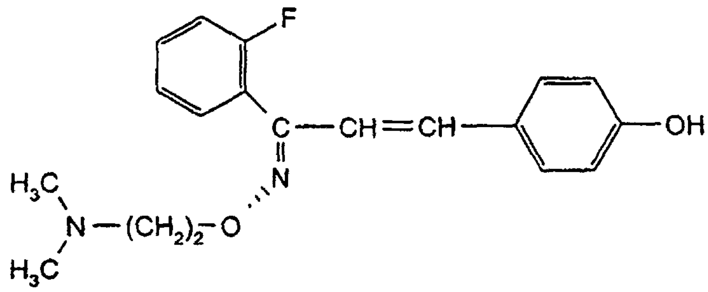

(I)

Plus particulièrement, l'hémifumarate de (1Z,2E)-1-(2-fluorophényl)-3-(4-hydroxyphényl)-prop-2-èn-1-one-O-(2-diméthylaminoéthyl)-oxime, connu sous le nom de code SR 46349 B et ci-après dénommé composé A, a été étudié pour ses propriétés biochimiques et pharmacologiques. Le composé A est un antagoniste spécifique du récepteur $5HT_{2A}$, à savoir qu'il n'a pas d'affinité pour les récepteurs $5HT_{1A}$, $5HT_{1B}$ et $5HT_{1D}$ et une affinité faible pour le récepteur $5HT_{2C}$ ; dans les études sur les tissus isolés, l'absence d'activité du composé A sur le fundus stomachal du rat indique une spécificité $5HT_{2A}$ versus $5HT_{2B}$ (M. Rinaldi-Carmona et al., J. Pharmacol. Exp. Ther., 1992, 262, 2, 759-768). Chez le rongeur, on a montré que ce composé se lie de façon majoritaire aux régions du cerveau contenant le récepteur $5HT_2$ (M. Rinaldi-Carmona et al., Life Sciences, 1993, 54, 119-127).

Des études sur le sommeil ont montré que certains antagonistes des récepteurs $5HT_2$ comme la ritansérine, l'amoxapine, et ICI 169 369, modifient l'architecture du sommeil et régulent ou augmentent le temps de sommeil lent (G. Loas, L'encéphale, 1991, XVII, 423-425).

Les mécanismes centraux par lesquels la sérotonine module l'activité respiratoire ont été étudiés et l'on a trouvé que parmi les différentes familles de récepteurs, seuls les récepteurs $5HT_1$ et les récepteurs $5HT_2$ affectent l'activité respiratoire au niveau central (R. Monteau et al., Eur. J. Pharmacol., 1994, 259, 71-74).

Dans le même article, ces auteurs étudient in-vitro sur des préparations de tissus de rats nouveaux-nés, à l'aide du composé A, quels sous-types de récepteurs interviennent dans la modulation de l'activité respiratoire. Ils observent que le prétraitement par le composé A empêche ou réduit de façon significative l'activité tonique cervicale induite par la 5-hydroxytryptamine et attribuée à l'activation des récepteurs spinaux $5HT_2$ ; de même, il inhibe l'effet dépresseur de la 5-hydroxytryptamine sur l'activité du nerf hypoglosse. Par ailleurs, les auteurs suggèrent que le composé A pourrait être utilisé pour l'étude in-vivo des mécanismes responsables de l'apnée obstructive.

L'utilisation du L-tryptophane, un précurseur de la sérotonine, dans les troubles respiratoires du sommeil a été étudiée (H.S. Schmidt, Bull. Eur. Physiopathol. Respir., 1983, 19, 625-629) ainsi que celle de la fluoxétine, inhibiteur sélectif de la recapture de la sérotonine (Hanzel D.A., Chest, 1991, 100, 416-421).

La demande de brevet européenne EP 449 561 A indique l'utilisation de la (R)-fluoxétine, pour traiter différentes affections dont les apnées du sommeil.

Un article de M. Yoshioka et al. dans J. Pharmacol. Exp. Ther., 1992, 260 (2), 917-924 concerne la caractérisation pharmacologique de l'apnée induite par la 5-HT chez le rat ; il rapporte que des antagonistes du récepteur 5-HT2 tels que la kétansérine et le méthysergide, inhibent l'apnée induite par la 5-HT, suggérant que l'apnée induite par la 5-HT est médiée par l'inhibition de l'activité nerveuse phrénique afférente.

Dans un article récent, S.C. Veasey at al. (Am. J. Respirat. Critic. Care Med., 1996, 153, 776-786) étudient les effets de deux antagonistes de la sérotonine sur un modèle animal de troubles respiratoires du sommeil : le bouledogue anglais. Ils concluent que la ritansérine et le méthysergide qui antagonisent notamment les récepteurs $5HT_2$ , administrés de façon systémique, conduisent à une diminution marquée de l'activité du muscle dilatateur des voies respiratoires supérieures et à une faible diminution de l'activité du diaphragme, ces diminutions coïncidant avec les désaturations en oxyhémoglobine. Les auteurs suggèrent que la sérotonine pourrait jouer un rôle dans l'augmentation de l'activité dilatatrice des voies respiratoires supérieures qui est connue pour intervenir dans le syndrome d'apnée du sommeil.

D. Rose et al. (Fundam. Clin. Pharmacol., 1996, 10 (1), 80) rapportent les résultats d'études réalisées in-vivo sur des animaux nouveaux-nés décérébrés (rats et chats). Chez le chat, ils observent que l'administration de fortes

doses de 5-hydroxytryptamine induit des apnées centrales prolongées liées à des périodes d'expiration active. Chez le rat, ils n'observent aucune apnée après administration de 5-hydroxytryptamine, ce qui est en contradiction avec les résultats observés in-vitro chez le rat nouveau-né.

Les différences interespèces observées, sur les mécanismes respiratoires ainsi que les différences entre les résultats des études in-vivo et in-vitro chez le rat ne donnent aucune indication à l'homme de l'art sur l'effet éventuel du composé (I) sur l'apnée du sommeil.

De façon inattendue, on a maintenant trouvé que le composé de formule (I), notamment le composé A, antagoniste des récepteurs $5HT_{2A}$ de la sérotonine, est efficace dans le traitement du syndrome d'apnée du sommeil.

Ainsi la présente invention est relative à l'utilisation d'un composé de formule (I) pour la préparation de médicaments utiles dans le traitement du syndrome d'apnée du sommeil, en particulier le syndrome d'apnée-hypopnée obstructive du sommeil.

Le syndrome d'apnée du sommeil est défini par un arrêt (apnée) ou une diminution (hypopnée) de la respiration survenant de façon récurrente au cours du sommeil. Les hypopnées et apnées interviennent à la fois pendant le sommeil paradoxal et pendant le sommeil lent. Les arrêts répétés de la respiration induisent une diminution de la saturation en oxyhémoglobine ($SaO_2$) et des éveils, conduisant à une fragmentation du sommeil et à la disparition des stades 3 et 4 correspondant au sommeil lent profond. Les conséquences cliniques de ce syndrome incluent :

i) une augmentation de la susceptibilité aux complications cardiovasculaires telles que l'hypertension pulmonaire, l'insuffisance cardiaque, l'hypertension, les arythmies cardiaques, les accidents vasculaires cérébraux et l'infarctus du myocarde ;

ii) une somnolence excessive pendant la journée et secondairement des risques d'accidents.

De plus, la fragmentation du sommeil et la désaturation nocturne en oxyhémoglobine entrainent fatigue, irritabilité, céphalées matinales, troubles de la mémoire, et/ou troubles de la personnalité.

Des données épidémiologiques récentes indiquent que ce syndrome affecterait 2 % à 4 % de la population adulte ou davantage ; les hommes et les obèses étant plus particulièrement touchés (New Engl. J. Med., 1993, 328 (17), 1230-1235).

A l'heure actuelle le principal traitement prescrit est, outre la perte de poids et la chirurgie, le traitement mécanique par ventilation en pression positive continue (ppc). Le principe de ce dernier traitement (ppc) repose sur l'administration par voie nasale d'une pression inspiratoire et expiratoire positive visant à supprimer le collapsus des voies aériennes supérieures. Ce traitement est encombrant, bruyant et contraignant, obligeant le patient à porter un masque nasal toutes les nuits ; de plus, il présente des effets secondaires tels que bouche sèche, éternuements, saignements de nez ou aérophagie. A ce jour, aucun traitement par un agent pharmacologique n'est connu (D.W. Hudgel, Chest, 1996, 109/5, 1346-1358).

On a maintenant trouvé que le composé de formule (I), notamment le composé A, est actif chez l'homme dans le traitement du syndrome d'apnée du sommeil.

Chez des sujets sains jeunes (18 à 35 ans), on a observé que l'administration du composé A induit un doublement de la durée des stades 3 et 4 du sommeil lent dès la dose de 1 mg ; les stades 1 et 2 du sommeil lent étant légèrement diminués et le sommeil paradoxal n'étant pas modifié.

L'effet du composé A sur des patients atteints d'apnée du sommeil est déterminé au cours d'une étude clinique réalisée en double aveugle versus placebo.

Les patients participant à cette étude ont un indice d'apnée-hypopnée supérieur à 25 et présentent également des symptômes tels que somnolence diurne, hypertension, fatigue, céphalée matinale, nycturie etc...

L'apnée est définie comme l'absence de flux aérien nasobuccal durant au moins 10 secondes. L'hypopnée est définie comme une réduction d'au moins 50 % du flux aérien nasobuccal pendant au moins 10 secondes. L'indice d'apnée-hypopnée (AHI) est le nombre d'apnées et d'hypopnées intervenant par heure. Cet indice est obtenu par un enregistrement polysomnographique mesurant également le débit ventilé, la pression oesophagienne et la saturation en oxyhémoglobine ($SaO_2$).

Une gélule contenant 5 mg du composé A est administrée avec le repas du soir, chaque jour pendant 14 jours. On observe une diminution nette de l'indice d'apnée-hypopnée chez les sujets traités.

Ainsi, la présente invention a pour objet l'utilisation d'un composé de formule (I) pour la préparation de médicaments utiles dans le traitement du syndrome d'apnée du sommeil.

Le composé de formule (I) et ses sels pharmaceutiquement acceptables sont préparés selon la description donnée dans le brevet européen 0 373 998 B1.

Dans les compositions pharmaceutiques pour l'administration orale, sublinguale, sous-cutanée, intramusculaire, intraveineuse, transdermique, locale ou rectale, le principe actif seul ou en association avec un autre principe actif peut être administré sous forme unitaire d'administration, en mélange avec des supports pharmaceutiques classiques, aux animaux et aux êtres humains. Les formes unitaires d'administration appropriées comprennent les formes

par voie orale telles que les comprimés, les gélules, les poudres, les granules et les solutions ou suspensions orales, les formes d'administration sublinguale et buccale, les aérosols, les implants, les formes d'administration sous-cutanée, intramusculaire, intraveineuse, intranasale et les formes d'administration rectale.

Dans les compositions pharmaceutiques, le principe actif est généralement formulé en unités de dosage. L'unité de dosage contient de 0,05 à 50 mg, avantageusement de 0,1 à 10 mg, de préférence de 0,5 à 5 mg d'antagoniste des récepteurs $5HT_{2A}$ par unité de dosage pour les administrations quotidiennes.

Lorsque l'on prépare une composition solide sous forme de comprimé, on peut ajouter au principe actif micronisé ou non, un agent mouillant et on mélange le tout avec un véhicule pharmaceutique tel que la silice, l'amidon, le lactose, le stéarate de magnésium, le talc ou analogues. On peut enrober les comprimés de saccharose, de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif ou les principes actifs avec un diluant en incorporant le mélange obtenu dans des gélules molles ou dures.

Une préparation sous forme de sirop ou d'élixir peut contenir le principe actif ou les principes actifs conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone ou polyvidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol ou le butylèneglycol.

Ainsi, pour préparer une solution aqueuse injectable par voie intraveineuse on peut utiliser un cosolvant : un alcool tel que l'éthanol, un glycol tel que le polyéthylèneglycol ou le propylèneglycol et un tensioactif hydrophile tel que le polysorbate 80. Pour préparer une solution huileuse injectable par voie intramusculaire, on peut solubiliser le principe actif par un triglycéride ou un ester de glycérol.

Pour l'administration transdermique, on peut utiliser des patches sous forme multilaminée ou à réservoir dans lequel le principe actif est en solution alcoolique.

Le principe actif peut être formulé également sous forme de microcapsules ou microsphères, éventuellement avec un ou plusieurs supports ou additifs.

Le principe actif peut être également présenté sous forme de complexe avec une cyclodextrine, par exemple $\alpha$-, $\beta$- ou $\gamma$- cyclodextrine, 2-hydroxypropyl-$\beta$-cyclodextrine ou méthyl-$\beta$-cyclodextrine.

Parmi les formes à libération prolongée utiles dans le cas de traitements chroniques, on peut utiliser des implants. Ceux-ci peuvent être préparés sous forme de suspension huileuse ou sous forme de suspension de microsphères dans un milieu isotonique.

Selon la présente invention, les formes orales d'administration sont préférées.

EXEMPLE 1 : Gélule à 0,1 mg de (1Z,2E)-1-(2-fluorophényl)-3-(4-hydroxyphényl)-prop-2-èn-1-one-O-(2-diméthylaminoéthyl)-oxime.

| | |
|---|---|
| Composé A | 0,118 mg |
| Lactose monohydrate extra-fin cristallisé | 99,132 mg |
| Amidon de maïs modifié | 25 mg |
| Silice colloïdale anhydre | 0,11 mg |
| Stéarate de magnésium | 0,64 mg |
| Pour une gélule blanc opaque de taille 0, terminée à | 125 mg |

EXEMPLE 2 : Gélule à 1 mg de (1Z,2E)-1-(2-fluorophényl)-3-(4-hydroxyphényl)-prop-2-èn-1-one-O-(2-diméthylaminoéthyl)-oxime.

| | |
|---|---|
| Composé A | 1,18 mg |
| Lactose monohydrate extra-fin cristallisé | 451,42 mg |
| Amidon de maïs modifié | 114 mg |
| Silice colloïdale anhydre | 0,5 mg |
| Stéarate de magnésium | 2,9 mg |
| Pour une gélule blanc opaque de taille 0, terminée à | 570 mg |

EXEMPLE 3: Gélule à 5 mg de (1Z,2E)-1-(2-fluorophényl)-3-(4-hydroxyphényl)-prop-2-èn-1-one-O-(2-diméthylaminoéthyl)-oxime.

| | |
|---|---|
| Composé A | 5,9 mg |
| Lactose monohydrate extra-fin cristallisé | 446,7 mg |
| Amidon de maïs modifié | 114 mg |
| Silice colloïdale anhydre | 0,5 mg |
| Stéarate de magnésium | 2,9 mg |
| Pour une gélule blanc opaque de taille 0, terminée à | 570 mg |

EXEMPLE 4: Gélule à 10 mg de (1Z,2E)-1-(2-fluorophényl)-3-(4-hydroxyphényl)-prop-2-èn-1-one-O-(2-diméthylaminoéthyl)-oxime.

| | |
|---|---|
| Composé A | 11,8 mg |
| Lactose monohydrate extra-fin cristallisé | 440,8 mg |
| Amidon de maïs modifié | 114 mg |
| Silice colloïdale anhydre | 0,5 mg |
| Stéarate de magnésium | 2,9 mg |
| Pour une gélule blanc opaque de taille 0, terminée à | 570 mg |

## Revendications

1. Utilisation du 1-(2-fluorophényl)-3-(4-hydroxyphényl)-prop-2-èn-1-one-O-2-(diméthylaminoéthyl)-oxime ou d'un de ses sels pharmaceutiquement acceptables pour la préparation de médicaments utiles dans le traitement du syndrome d'apnée du sommeil.

2. Utilisation selon la revendication 1 de l'hémifumarate de (1Z, 2E)-1-(2-fluorophényl)-3-(4-hydroxyphényl)-prop-2-èn-1-one-O-(2-diméthylaminoéthyl)-oxime.

3. Utilisation du 1-(2-fluorophényl)-3-(4-hydroxyphényl)-prop-2-èn-1-one-O-2-(diméthylaminoéthyl)-oxime ou d'un de ses sels pharmaceutiquement acceptables pour la préparation de médicaments utiles dans le traitement du syndrome d'apnée-hypopnée obstructive du sommeil.

4. Utilisation selon la revendication 3 de l'hémifumarate de (1Z, 2E)-1-(2-fluorophényl)-3-(4-hydroxyphényl)-prop-2-èn-1-one-O-(2-diméthylaminoéthyl)-oxime.

**Patentansprüche**

1. Verwendung von 1- (2-Fluorphenyl)-3-(4-hydroxyphenyl)-prop-2-en-1-on-O-2-(dimethylaminoethyl)-oxim oder eines seiner pharmazeutisch annehmbaren Salze für die Herstellung von Arzneimitteln zur Behandlung des Schlafapnoe-Syndroms.

2. Verwendung nach Anspruch 1 des Hemifumarats von (1Z, 2E)-1-(2-Fluorphenyl)-3-(4-hydroxyphenyl)-prop-2-en-1-on-O-(2-dimethylaminoethyl)-oxim.

3. Verwendung von 1-(2-Fluorphenyl)-3-(4-hydroxyphenyl)-prop-2-en-1-on-O-2-(dimethylaminoethyl)-oxim oder eines seiner pharmazeutisch annehmbaren Salze für die Herstellung von Arzneimitteln zur Behandlung des obstruktiven Schlaf-Apnoe-Hypopnoe-Syndroms.

4. Verwendung nach Anspruch 3 des Hemifumarats von (1Z, 2E)-1-(2-Fluorphenyl)-3-(4-hydroxyphenyl)-prop-2-en-1-on-O-(2-dimethylaminoethyl)-oxim.

**Claims**

1. Use of 1-(2-fluorophenyl)-3-(4-hydroxyphenyl)-prop-2-en-1-one-O-(2-dimethylaminoethyl)oxime, or of one of its pharmaceutically acceptable salts, for the preparation of medicines useful in the treatment of sleep apnoea syndrome.

2. Use, according to Claim 1, of the hemifumarate of (1Z, 2E)-1- (2-fluorophenyl)-3- (4-hydroxyphenyl)prop-2-en-1-one-O-(2-dimethylaminoethyl)oxime.

3. Use of 1-(2-fluorophenyl)-3-(4-hydroxyphenyl)-prop-2-en-1-one-O-(2-dimethylaminoethyl)oxime, or of one of its pharmaceutically acceptable salts, for the preparation of medicines useful in the treatment of sleep obstructive apnoea-hypoapnoea syndrome.

4. Use, according to Claim 3, of the hemifumarate of (1Z, 2E)-1-(2-fluorophenyl)-3-(4-hydroxyphenyl)prop-2-en-1-one-O-(2-dimethylaminoethyl)-oxime.